# EUROPEAN PATENT APPLICATION

(11) **EP 1 614 697 A1**
(43) Date of publication of application: **11.01.2006**
(21) Application number: 04720755.0
(22) Date of filing: 15.03.2004
(51) Int. Cl.: C08B 37/08, A61K 38/55, A61P 17/06, A61P 19/02, A61P 27/02, A61P 27/06, A61P 29/00, A61P 35/00, A61P 43/00, C07K 14/46, C12P 21/00

(54) **PROTEOGLYCAN ISOLATED FROM CARTILAGINOUS FISH AND PROCESS FOR PRODUCING THE SAME**

(30) Priority: 20.03.2003 JP 2003077778
(71) Applicant: HOSOKAWA MICRON CORPORATION, Osaka-Shi, Osaka 541-0048 (JP)
(72) Inventor: SATO, Kenji, Rm. 102, Lions Manshion Kitayamadori, Kyoto-shi, Kyoto 603-8206 (JP); TSUTSUMI, Masahiro, Kashiba-shi, Nara 639-0254 (JP); NOMURA, Yoshihiro, Fuchu-shi, Tokyo 183-0046 (JP); MURATA, Nao, Kashihara-shi, Nara 634-0831 (JP); KONDO, Naoyuki, Hosokawa Micron Corporation, Osaka-shi, Osaka 541-0048 (JP)
(74) Representative: Vossius & Partner
(86) International application number: PCT/JP2004/003432
(87) International publication number: WO 2004/083257

(57) **Abstract**

There are provided a cartilage extract that can be effective even when taken in a small amount and has a clear mechanism of effective action and a method of producing the extract. Provided are an isolated proteoglycan which is derived from a water extract of cartilage of cartilaginous fish and whose main component has a molecular weight of 500 kDa or more; a composition containing the proteoglycan; a pharmaceutical composition containing the proteoglycan as an active ingredient; and a method of producing the proteoglycan, comprising the steps of pulverizing cartilaginous fish-derived cartilage into a pulverized product with an average particle diameter of 100 µm or less, adding water to the pulverized product and extracting water-soluble components from it, separating an aqueous phase that contains the extracted water-soluble components, and adding an alcohol to the aqueous phase to produce a precipitate.

SEQUENCE LISTING

<110>  HOSOKAWA MICRON CORPORATION

<120>  An Isolated Proteoglycan Derived from Chondrichthyes and Method for
Producing Thereof

<130>  L 2405 EP

<140>  PCT/JP2004/003432
<141>  2004-03-15

<150>  JP/2003/77778
<151>  2003-03-20

<160>  2

<170>  PatentIn version 3.1

<210>  1
<211>  14
<212>  PRT
<213>  Hamster sp.

<400>  1

Val Asp Leu Ser Asn Asp Ala Met Asp Thr Ala Asp Asp Ser
1               5                   10


<210>  2
<211>  14
<212>  PRT
<213>  Hamster sp.

<400>  2

Ile Ile Gly Gly Ser Leu Asp Ala Lys Gly Ser Phe Pro Trp
1               5                   10

## Description

### TECHNICAL FIELD

The invention relates to a proteoglycan isolated from cartilaginous fish and to a method of producing the same. More specifically, the invention relates to a proteoglycan that can be useful for health maintenance or an improvement in quality of life or useful as a pharmaceutical and to a method of producing such a proteoglycan.

### BACKGROUND ART

Cancer is a common troublesome human disease, and how to cure it is becoming a global issue. Particularly in Japan, cancer has become the leading cause of death, while the major cause of death has been changed from infectious diseases to lifestyle-related diseases, and therefore, cancer has been investigated as the highest priority issue on diseases control. Cancer can occur at any organ or tissue depending on constitution, sex, age, place, and lifestyle. Every features including metastasis and growth potential depend on the type of cancer, and thus under present circumstances, a decisive therapy has not been established yet.

The characteristics of cancer cells include vascularization for nutrient uptake, unregulated autonomous proliferation, and production of new cancerous tissues at various parts of the body by invasion or metastasis. In addition, cancerous cachexia debilitates the host, usually killing it. Examples of current cancer therapy include excision, administration of anticancer agents, immunotherapy, thermotherapy, and exposure to radiation. However, the therapies vary in their efficacy with the type of cancer, and some therapies are not available depending on primary lesion. The weakness of the host due to side effect is also a problem. Thus, attention has focused on therapeutic methods to inhibit any of vascularization, invasion and metastasis, which are common to solid tumors (Folkman J., Nat. Med. 1:27-31, 1995).

It is known that for invasion, metastasis and vascularization, cancer cells mainly use matrix metalloprotease (MMP) to decompose extracellular matrixes or basement membranes surrounding the cells. Based on this information, against such a new target, current cancer therapy starts to use a method in which MMP is inhibited so that invasion or metastasis of cancer and vascularization can be inhibited and that cancer progression can also be inhibited, and a variety of synthetic MMP inhibitors have been developed (Iki K. et al., Carcinogenesis, 1999 Jul;20(7):1323-9; Rasmussen A. H. et al., International Business Communications (1997); Naito K. et al., Int J Cancer 1994 Sep 1;58(5):730-5). However, bone-marrow toxicitymyelotoxicity, cytotoxity, and arthralgia reported as their side effects have raised practical problems.

In current trends in natural products, materials effective in preventing various diseases or in inhibiting progression of various diseases have been found in traditional Chinese medicine materials or food materials, and part of them have been certified as "food for specified health use" for indication of their efficacy by the Ministry of Health, Labor and Welfare. In the field of cancer therapy, attention has focused on food material-derived components, and various food components have been investigated for their cancer-inhibiting effects. Among such food materials, blood vessels are not formed in cartilage tissues, and thus it is believed that cartilage tissues should contain a substance with a vascularization-inhibiting activity. In fact, inhibition of tumor growth or vascularization was observed in an experiment in which a cartilage extract was injected into the periphery of tumors (Lee A. et al., Science 1983 Sep 16;221(4616):1185-7). Inhibition of MMP is considered as an ability of shark cartilage to inhibit vascularization, and inhibition of MMP was found in vitro (Moses, M. A. et al., J. Cell. Biochem. 47:230-235, 1991; Gingras D. et al., Anticancer. Res. 2001:19(1-2):83-6). It is also reported that inhibition of vascularization was observed in an experiment in which shark cartilage was orally administered to animals (Davis PF. et al., Microvasc Res 1997 Sep;54(2): 178-82). Based on these findings, alternative therapies or clinical studies have been performed in which shark cartilage powder is orally administered to cancer patients in expectation of retardation or inhibition of cancer progression, because more cartilage of better quality can be collected from sharks, cartilaginous fish, than from bony animals (Ernst E., Lancet 351:298 1998). However, the in vivo mechanism of the anti-tumor effect of orally administered shark cartilage is almost unknown. It is also unknown whether shark cartilage can inhibit MMP in tissues when orally administered.

Known methods of obtaining active components from shark cartilage include a method comprising micronizing shark cartilage and directly using the micronized product as an active component (JP-A No. 2001-48795) and a method comprising crushing shark cartilage, extracting the crushed product with water and freeze-drying the extract (Japanese Patent Application National Publication No. 09-512563, U.S. Patent No. 4,473,551).

### DISCLOSURE OF INVENTION

However, the shark cartilage-derived components produced by the above methods must be taken in a large amount to develop the effect or must be used in combination with another active ingredient so that anti-tumor activity or anti-inflammatory activity can be achieved, and the mechanism of the effect has not been proved.

Thus, it is an object of the invention to provide a cartilage extract that can be effective when taken in small amounts and can have a clear mechanism of effective action and to provide a method of producing such a cartilage extract.

In order to achieve the object, the inventors have made active investigations on the conditions for efficient extraction of active ingredients from cartilage and on the properties of the active ingredients and finally found that the desired object can be achieved by a component satisfying the requirements below and by a method of producing such a component in completing the invention.

Thus, the isolated proteoglycan of the invention is characterized that it is derived from a water extract of cartilage of cartilaginous fish and whose main component has a molecular weight of 500 kDa or more.

The proteoglycan is preferably insoluble in an alcohol.

The proteoglycan preferably has a glycosaminoglycan part mainly composed of chondroitin sulfate C.

The proteoglycan preferably has a matrix metalloprotease-inhibiting activity.

When the matrix metalloprotease is MMP-9, the inhibiting activity is preferably an effect of canceling a reduction in an MMP-9-inhibiting activity in the blood serum of a tumor-bearing animal fed on a 0.4% by weight-product-containing feed or an effect of increasing, by at least 5%, an MMP-9-inhibiting activity in the blood serum of a tumor-bearing animal fed on a 0.4% by weight-product-containing feed.

The proteoglycan preferably has an effect of increasing a cathepsin B-inhibiting activity when taken in an effective amount into a living body.

The proteoglycan preferably has an activity of increasing the amount of haptoglobin in blood serum when taken in an effective amount into a living body.

The composition of the invention is characterized by comprising the proteoglycan.

The composition is preferably for use in an improvement in quality of life.

The pharmaceutical composition of the invention is characterized by comprising the proteoglycan as an active ingredient.

The method of producing a proteoglycan of the invention is characterized by comprising the steps of:
pulverizing cartilaginous fish-derived cartilage into a pulverized product with an average particle diameter of 100 µm or less;
adding water to the pulverized product and extracting water-soluble components from it;
separating an aqueous phase that contains the extracted water-soluble components; and
adding an alcohol to the aqueous phase to produce a precipitate.

### BRIEF DESCRIPTION OF DRAWINGS

Fig. 1 is a chart showing the composition of a water extract of shark cartilage obtained in Example 1;
Fig. 2 is a chart showing the amino acid composition of an MMP-9-inhibiging activity fraction obtained by gel filtration HPLC in Example 1;
Fig. 3 is a chart showing MMP-2- and MMP-9-inhibiting activities of the water extract of shark cartilage obtained in Example 1;
Fig. 4 is a chart showing a correlation between pH and MMP-9 inhibiting activity for the respective isoelectric focusing fractions;
Fig. 5 is a chart of gel filtration (Superdex® 75);
Fig. 6 is an electrophoretogram showing the results of examination of MMP-9-inhibiting activity for the respective gel filtration (Superdex® 75) fractions;
Fig. 7 is a chart showing a result of cellulose acetate film electrophoresis;
Fig. 8A is a chart of gel filtration (Superdex® 200);
Fig. 8B is a chart showing the relationship between elution times on Superdex® 200 and molecular weights;
Fig. 8C is a chart showing MMP-9-inhibiting activities for the respective fractions obtained through Superdex® 200;
Fig. 9 is a chart showing a comparison between the MMP-9-inhibiting activities of a product according to the invention, chondroitin sulfate C, and commercially available shark cartilage, wherein it shows a remaining activity of MMP-9 after 5 µl of an aqueous solution of a sample is added to 10 µl of an active form of MMP-9 and allowed to react at 37°C for 24 hours;
Fig. 10 is a chart showing cancer-inhibiting effects in chemically-caused-cancer-bearing hamsters in Example 1, wherein it shows the number of tumors after 50-day feeding with basal diet or experimental diet (the star mark: p<0.05);
Fig. 11 is a chart showing MMP-9-inhibiting activity in the blood serum of a pancreatic cancer patient in Example 3; and
Fig. 12 is a chart showing the results of quantitative determination of type IV collagen fragments in the blood serum of a pancreatic cancer patient in Example 3.

### BEST MODE FOR CARRYING OUT THE INVENTION

The isolated proteoglycan of the invention is derived from a water extract of cartilage of cartilaginous fish and comprises a main component having a molecular weight of 500 kDa or more.

Any species of cartilaginous fish may be used as a raw material for the proteoglycan without limitation and includes Elasmobranch such as shark and ray and Holocephali such as chimaera.

In the invention, any cartilage derived from the cartilaginous fish may be used without limitation.

The term "main component" refers to a component that can show a main peak at a fraction with a molecular weight of 500 kDa or more by gel filtration chromatography.

Thus, "a molecular weight of 500 kDa or more" refers to a molecular weight estimated by gel filtration chromatography, which is a molecular weight specifically measured with Superdex® 200 in the range of from 500 kDa to the exclusion limit 1300 kDa.

The proteoglycan of the invention is preferably insoluble in an alcohol. The alcohol is preferably a lower alcohol such as methanol, ethanol, and isopropanol.

Specifically, the proteoglycan of the invention is preferably an alcohol-insoluble macromolecular compound as specified above.

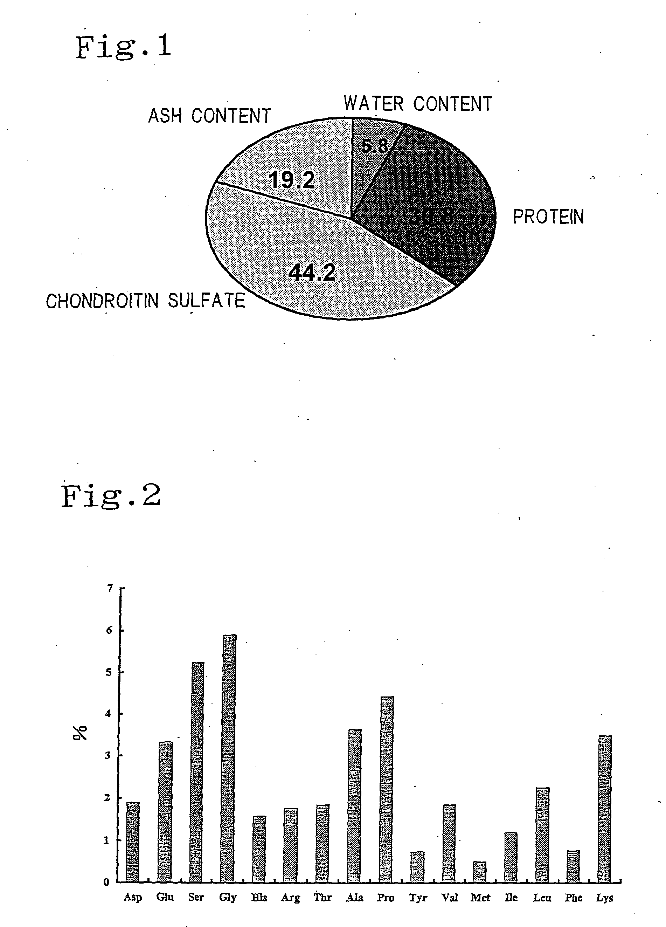

Herein, the proteoglycan refers to a compound of glycosaminoglycan covalently bonded to protein. The glycosaminoglycan part preferably comprises chondroitin sulfate C as a main component. The protein part preferably has the amino acid composition as shown in Example 1 and Fig. 2, described later.

The proteoglycan of the invention preferably has a matrix metalloprotease (MMP)-inhibiting activity. In a preferred mode, the proteoglycan has an effect of increasing the matrix metalloprotease (MMP)-inhibiting activity when taken in an effective amount into a living body.

The MMP may be any animal-derived protease without limitation. Examples of the animal include rodents such as mouse, rat, and hamster, domestic animals such as chicken, rabbit, cow, sheep, pig, and horse, and human. Examples of the type of MMP include inducible MMPs such as MMP-1, MMP-3, MMP-7, MMP-9, MMP-11, MMP-12, and MMP-13; and constant MMPs such as MMP-2 and MMP-14. MMP-2 or MMP-9 is preferred, because it is involved in tumor vascularization and belongs to a gelatinase group capable of decomposing type IV or V collagen which is a component of basement membranes for supporting blood vessels.

In the invention, "an effective amount" refers to an amount sufficient for the production of the effect of increasing the MMP-inhibiting activity in a living animal, the production of the effect of increasing the cathepsin B-inhibiting activity in a living animal or the production of the effect of increasing the amount of haptoglobin in the blood serum of a living animal. The effective amount may vary with animal's individual characteristics such as the kind, age, gender, and weight of the animal. For example, the effective amount may be from about 1 to about 50 mg per day for an increase in the MMP-inhibiting activity in a hamster or from about 1 to about 15 g per day for an increase in the MMP-inhibiting activity in a human being.

When the MMP is MMP-9, the increase in the MMP-9-inhibiting activity is preferably an effect of canceling a reduction in the MMP-9-inhibiting activity in the blood serum of a tumor-bearing animal fed on a 0.4% by weight-proteoglycan-containing feed or an effect of increasing, by at least 5%, the MMP-9-inhibiting activity in the blood serum of a tumor-bearing animal fed on a 0.4% by weight-proteoglycan-containing feed. In a more preferred mode, the MMP-9-inhibiting activity increases by 20% or more. Such an effect can result in effective suppression of the growth or metastasis of cancer.

Measurement of the MMP-9-inhibiting activity in blood serum has been made possible for the first time by the inventors. The MMP-9-inhibiting activity in blood serum can be measured by analyzing a mixture of specific amounts of type V collagen and MMP-9 with blood serum by SDS-PAGE and Western blotting and then determining the amount of type V collagen by densitometry. Concerning the activity of MMP-9, an activity of decomposing 10% of type V collagen by the reaction at 37°C for 24 hours is defined as 1 mU. Accordingly, concerning the MMP-9-inhibiting activity in blood serum, an activity of inhibiting 1 mU of MMP-9 activity may be defined as 1 mU.

In a preferred mode, the proteoglycan of the invention has an effect of increasing the cathepsin B-inhibiting activity when taken in an effective amount into a living body. An anti-cancer effect can be produced through the cathepsin B-inhibiting effect. The types of cathepsin B include those derived from the same animal species as for the MMPs.

According to the method described in Methods of Enzymology 1981;vol.80, p. 535-561, the cathepsin B-inhibiting activity may be measured using the blood serum of a living animal ingesting it. Whether the activity is significantly higher in blood serum after the intake of the proteoglycan of the invention than that in the blood serum before the intake may be used as an indicator.

In a preferred mode, the proteoglycan of the invention has an activity of increasing the amount of haptoglobin in blood serum when taken in an effective amount into a living body. If the amount of haptoglobin in blood serum is increased, an anti-cancer effect can be produced through the cathepsin B-inhibiting effect. The types of haptoglobin include those derived from the same animal species as for the MMPs.

The amount of haptoglobin in the blood serum may be determined by subjecting the blood serum of a living animal ingesting it to SDS-PAGE and then staining the blood serum and determining it with a densitometer. Whether the activity is significantly higher in blood serum after the intake of the proteoglycan of the invention than that in the blood serum before the intake may be used as an indicator.

According to the invention, there is provided a composition comprising the proteoglycan. While the composition may have any proper proteoglycan content depending on purpose, the proteoglycan content of the composition is generally from 0.001 to 99.9% by weight.

Other ingredients may be properly selected for use in the composition depending on purpose. For example, the composition for use as a food product may be prepared by mixing a certain amount of the proteoglycan of the invention with general food materials. The composition for use as a supplementary food product (so called a supplement) may be prepared by forming a mixture of the proteoglycan of the invention with a desired material such as a filler such as starch, a sweetener such as glucose, sucrose, and a syrup, a flavor, or water in a digestible form such as in the form of a tablet, a capsule, or a liquid. The composition may contain any component derived from cartilage of cartilaginous fish, such as pulverized cartilage itself (including a homogenate) and other components derived from a water extract of cartilage (such as a proteoglycan with a molecular weight of less than 500 kDa, chondroitin sulfate, hyaluronic acid, collagen, and minerals) or may contain any other component not derived from cartilage of cartilaginous fish but regarded as healthful, such as royal jelly, oyster extract, vitamins, glucosamine, chitin, chitosan, and yeast.

The composition contains the proteoglycan which can perform a specific function when taken into a living body and thus is preferably used for improvements in quality of life including maintenance and promotion of health and amelioration of illness (including diseased conditions).

According to the invention, there is provided a pharmaceutical composition comprising the proteoglycan as an active ingredient. Examples of diseases to be treated with the pharmaceutical composition of the invention include, but are not limited to, diseases accompanied by vascularization or angiogenesis such as cancer growth, cancer metastasis, arthritis such as rheumatoid arthritis, diabetic retinopathy, neovascular glaucoma, psoriasis, and inflammatory diseases accompanied by blood vessel components.

The pharmaceutical composition of the invention may be prepared directly with the proteoglycan or prepared by mixing the proteoglycan with any known pharmaceutically acceptable carrier (such as a vehicle, a filler, a binder, and a lubricant) or any conventional additive. The pharmaceutical composition may be orally or parenterally administered depending on its form of preparation (e.g. an oral administration form such as a tablet, a pill, a capsule, a powder, a granule, and a syrup, and a paranteral administration form such as an injection, a drip, an external preparation, and a suppository). While the dose depends on the content of the active ingredient, the administration route, the age, weight, or condition of the administration subject or the patient and cannot uniquely be defined, the composition is generally administered in a daily dose of several mg to about 20 g once to several times a day.

According to the invention, there is provided a method of producing the proteoglycan. The method is characterized by including the steps of:
pulverizing cartilaginous fish-derived cartilage into a pulverized product with an average particle diameter of 100 µm or less;
adding water to the pulverized product and extracting water-soluble components from it;
separating an aqueous phase that contains the extracted water-soluble components; and
adding an alcohol to the aqueous phase to produce a precipitate. A description is provided below of the process of the invention including the respective steps.

### (1) Materials and Pretreatment Thereof

Any type of cartilage derived from the cartilaginous fish may be used as a raw material without limitation. Unnecessary parts such as meat and skin binding around cartilage are preferably removed in advance. Any part of cartilage may be used such as middle cartilage, fin, and head cartilage, without limitation. While any of raw cartilage, dried cartilage, and frozen products thereof may be used as cartilage, dried cartilage or frozen products thereof is preferred in terms of availability of the material. Cartilage may be dried using sun drying or a drier at about 60°C.

### (2) Step of Pulverizing Cartilage into Pulverized Product with Average Particle Diameter of 100 µm or Less

The cartilage is pulverized for efficient performance of the extracting step below. The method of pulverization may be dry grinding or wet grinding. Dry grinding may be performed using dried cartilage or any frozen product thereof. Wet grinding may be performed using raw cartilage alone, using raw, or dried cartilage and water added thereto. Dry grinding is preferred in view of easiness of control of particle diameters of the pulverized product.

The apparatus for use in dry grinding may be a freeze crusher, a jet mill, or the like.

The apparatus for use in wet grinding may be a homogenizer, a wet medium mill, or the like.

The pulverized product may have an average particle diameter of from 0.1 to 100 µm, preferably from 1 to 50 µm. An average diameter of less than 0.1 µm can tend to reduce the productivity, and an average diameter of more than 100 µm can tend to reduce the efficiency of the extraction.

Herein, the "average particle diameter" refers to the diameter of a sphere having the same volume as a particle with the median of the volume-based distribution (the particle diameter at 50% of the integral distribution) and may be determined using a particle size distribution analyzer based on laser diffraction method, centrifugal sedimentation method, or the like.

In order to increase the productivity, cartilage may be coarsely crushed beforehand and then pulverized as described above. Any method and apparatus may be used for course crushing without limitation. For example, it may be performed using a coarse crusher such as a hammer mill. Course crushing may be performed so as to produce a crushed product with an average particle diameter of about 0.5 to about 10 mm.

### (3) Step of Adding Water to the Pulverized Product and Extracting Water-Soluble Components

Water is added to the pulverized product. Any type of water may be added such as tap water, ion-exchanged water, and distilled water. The water may have any temperature or any pH. Water may be used without specific pH control.

The water is generally added in an amount of 1 to 10 times the weight of the pulverized product.

The extraction conditions may include stirring the pulverized product and water, and performing extraction for a time period of generally from about 1 minute to about 24 hours. A time period of less than 1 minute can provide insufficient extraction. If the time period is not less than 24 hours, the productivity tends to be reduced. The extraction may be performed once or twice or more. The addition amount of the water and the number of extractions may be set such that the resulting aqueous phase can have a volume within a specific range.

### (4) Step of Separating Aqueous Phase Containing the Extracted Water-Soluble Components

After the extracting step is completed, an aqueous phase containing the extracted water-soluble components is separated from the extracted suspension. The method of separation may be general solid-liquid separation such as centrifugal separation, filtration, and decantation.

### (4') Concentration Step

The aqueous phase obtained by Step (4) may be concentrated as needed. Any method may be used for concentration, for example, including concentration by heating concentration, freeze concentration, ultrafiltration membrane concentration, and the like. The degree of the concentration may be set at any proper value depending on the volume of the aqueous phase but is generally 75% or less of the original volume.

### (5) Step of Adding Alcohol to the Aqueous Phase to Produce Precipitate

An alcohol is added to the aqueous phase resulting from Step (4) or (4') to precipitate alcohol-insoluble components so that a precipitate is obtained.

The alcohol to be added may be methanol, ethanol, or isopropanol. Ethanol is preferred for food product purposes. The alcohol may be added in such an amount that the precipitation of the insoluble components can be visually observed but is generally added in an amount of 1 to 3 parts by volume based on 1 part by volume of water. The precipitation conditions include, but are not limited to, standing at a temperature of 0 to 60°C for about 1 minute to about 24 hours.

### (6) Step of Recovering the Precipitate

The precipitate resulting from Step (5) may be recovered by centrifugal separation, filtration, or the like. The recovered precipitate is preferably dried in order to remove the alcohol and water. The drying method may be, for example, air-drying, freeze-drying, or the like.

### (7) Step of Particle Size Regulation of the Precipitate

In a preferred mode, the resulting precipitate is further subjected to water removal and powder preparation process so as to have good storage stability or handleability. Its dry water content may be such a degree that it can easily be stored. Pulverization, granulation, or the like may further be performed depending on use.

### (8) Step of Isolating Fractions with Molecular Weight of 500 kDa or More

The precipitate recovered by Step (6) consists essentially of the proteoglycan of the invention. The precipitate resulting from Step (6) may further be purified by gel filtration or the like depending on use. The method of purification may include isolating fractions with a molecular weight of 500 kDa or more by preparative gel filtration chromatography (for example, using Superdex® 200). In the concentration step, membrane filtration with a cut-off value of 500 kDa may be performed in place of Step (8).

### (9) Step of Isolating Fractions with a pI Value of 5.5 or Less by Isoelectric Focusing

The precipitate recovered by Step (6) or the product purified by Step (8) consists essentially of the proteoglycan of the invention. The precipitate resulting from Step (6) or the purified product resulting from Step (8) may be further purified by isoelectric focusing. The method of purification may include isolating fractions with a pI value of 5.5 or less by the use of a preparative isoelectric focusing apparatus (for example, Rotofor manufactured by Bio-Rad Laboratories Inc.). Isolated fractions preferably have a pI value of 5.0 or less, more preferably 2 to 3.

The fractions isolated by Step (8) or (9) may be subjected to solvent removal and then subjected to the particle size regulation of Step (7).

The resulting proteoglycan of the invention may be used alone or used as an active ingredient of food products or pharmaceuticals. When taken in an effective amount into an animal body, the proteoglycan of the invention can contribute to improvements in quality of life (QOL) including prevention and treatment of diseases through the effect of increasing the MMP-inhibiting activity, the effect of increasing the cathepsin B-inhibiting activity, or the effect of elevating the haptoglobin expression.

### Examples

The features and advantages of the invention are more specifically described by means of the examples and the like below, which are not intended to limit the scope of the invention.

### Example 1

### Isolation of Proteoglycan Derived from Shark Cartilage

### (1) Preparation of Shark Cartilage Extract

Sun-dried shark cartilage (cartilage of blue shark) was coarsely ground into particles with an average particle diameter of about 5 mm by means of a coarse crusher (FM-1 manufactured by Hosokawamicron Corporation). The resulting coarse ground product was pulverized together with liquid nitrogen in a freeze crusher (LX-1 manufactured by Hosokawamicron Corporation) to form a fine powder with an average particle diameter of 25 µm. The average particle diameter was measured using Microtrac 9320HRA (manufactured by Leeds and Northrup). To 3 kg of the resulting fine powder was added 15 kg of distilled water and stirred at room temperature for 20 minutes, and then centrifuged (5 minutes, 3,000 rpm) so that a supernatant was obtained. To the residue was further added 10 kg of distilled water and stirred at room temperature for 20 minutes and then centrifuged in the same manner so that a supernatant was obtained. The supernatants were combined and concentrated to an about 65% volume by means of an ultrafiltration membrane (SIP-1053, ID 1.4mm, manufactured Asahi Kasei Corporation, with a molecular weight cut-off of 6 kDa, ID 1.4 mm). Three parts by volume of ethanol was added to one part by volume of the resulting liquid concentrate and allowed to stand at room temperature for 2 hours so that a precipitate was produced. The precipitate was recovered by centrifugation (5 minutes, 3,000 rpm) and dried at 40°C. The dried extract of shark cartilage was ground in a coffee mill and used in the experiments below.

### (2) Measurement of Water Content

The water content was determined according to the heat-drying method at atmospheric pressure. The tare weight of a sufficiently dried crucible was measured, and 1 g of the sample obtained in Section (1) was precisely weighed in the crucible. The sample was dried at 120°C for 1 hour in a sample drier, allowed to stand to cool, and then weighed. This process was repeated until a constant weight was achieved. The water content of each sample was then calculated from weight loss (Fig. 1).

### (3) Measurement of Ash Content

The tare weight of a crucible was measured, and 0.2 g of the sample obtained in Section (1) was weighed in the crucible. The crucible was heated with a burner, and the heat was turned off when smoke rising terminated. The crucible was then transferred into a desiccator and placed in an electric furnace set at 500°C. After allowed to stand for 24 hours, the crucible was allowed to stand to cool and weighed. The ash content was calculated from the weight change (Fig. 1).

### (4) Amino Acid Analysis

100 µl of an aqueous solution of the sample obtained in Section (1) (200 µg/ml) and 30 µl of a standard sample (10 µl of a standard amino acid mixture solution (type H), 10 µl of 2.5 pmol/ml Hyp, and 10 µl of 2.5 pmol/ml Hyl) were each independently added to a cleaned small test tube (6 mm x 5 cm), centrifuged, and evaporated to dryness under vacuum. Each sample was then placed in a 15 ml reactive vial equipped with a mininert valve and hydrolyzed with 300 µl of 6 N HCl under reduced pressure (0.1 to 0.2 Torr) at 150°C for 1 hour. The sample was then evaporated to dryness under vacuum for removal of HCl.

10 µl of a prepared liquid mixture of methanol:triethylamine:distilled water=7:2:1 was added to each sample and evaporated to dryness under vacuum. 20 µl of a liquid derivatizing agent of methanol:triethylamine:distilled water:phenylisothiocyanate=7:1:1:1 was then added to each sample. Each sample was derivatized at room temperature for 20 minutes and then evaporated to dryness under vacuum. A 10% acetonitrile-containing 5 mM phosphate buffer (Pico-Tag sample diluent, pH 7.4, manufactured by Waters Corporation) was added in an amount of 500 µl to the standard and in an amount of 200 µl to the sample. Each test tube was sealed with Parafilm, and the phenylisothiocyanate crystal was ultrasonically disintegrated. After filtration with a 0.45 µm filter, each sample was centrifuged (12,000 rpm, 3 minutes), and the resulting supernatant (10 µl of the standard or 20 µl of the sample) was subjected to reverse-phase HPLC analysis using a column of Superspher 100 PR-18(e) (Merck Ltd.) and eluting solvent A: a 150 mM ammonium acetate buffer (pH 6.0) containing 5% acetonitrile and eluting solvent B: 60% acetonitrile. In the gradient composition, eluting solvent B was used as follows: 0 to 2 minutes: 0%, 2 to 20 minutes: 10 to 47.5%, 20 to 25 minutes: 47.5 to 100%, 25 to 37 minutes: 100%, 37 to 50 minutes: 0%. The detection was performed at 254 nm, a flow rate of 0.8 ml/min and a column temperature of 40°C. The results are shown in Fig. 1.

### (5) Measurement of the Amount of Chondroitin Sulfate by

### Carbazole-Sulfuric Acid Method

To a test tube was added 0.25 ml of an aqueous solution of the sample obtained in Section (1) (200 µg/ml). While the solution was cooled with ice water and stirred, 1.5 ml of a sulfuric acid solution (a solution of 0.95 g sodium tetraborate decahydrate in 100 ml concentrated sulfuric acid) was added dropwise thereto, and 0.05 ml of a 0. 125% carbazole solution (a solution of 12.5 mg carbazole in 10 ml ethanol) was added, and mixed sufficiently. The tube was then capped with a glass ball and heated for 20 minutes in boiling water. After cooled to room temperature, the sample was measured for absorbance at 530 nm with a spectrophotometer (SPECTROPHOTOMETER DU640, manufactured by Beckman) (T Bitter, H. M. Muir, Anal. Biochem. 4, 330, 1962). The same process was performed using chondroitin sulfate C (20, 40, 100, 200 µg/ml) as a standard.

### Results

As shown in Fig. 1, the water extract of shark cartilage obtained in Section (1) was mainly composed of protein (30.8%) and chondroitin sulfate (44.2%), and had a water content of 5.8% and an ash content of 19.2%. The protein in the shark cartilage extract had a Gly-rich amino acid composition and contained hydroxyproline (Hyp) and hydroxylysine (Hyl), which are typical of collagen. This composition was in substantial agreement with the composition of type II collagen of shark cartilage. The composition containing type II collagen and a relatively large amount of Ser and the like suggested that it should contain a protein component of proteoglycan.

A fraction having MMP-9-inhibiting activity was obtained by gel filtration purification as described later and then subjected to amino acid analysis in the same manner as in Section (4). The results are shown in Fig. 2, which suggested that the fraction with very little hydroxyproline should comprise a protein component mainly derived from proteoglycan.

### (6) MMP-2 or MMEP-9-Inhibiting Activity of Water Extract of Shark Cartilage

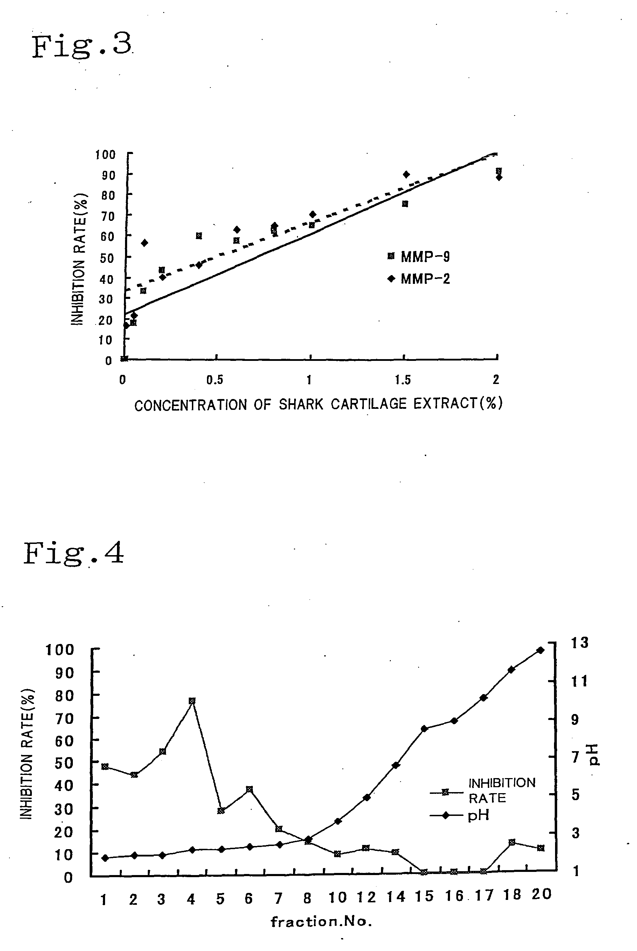

To a 0.6 ml microtube were added 10 µl of a 100 mM Tris-HCI buffer (pH 7.86) containing 400 mM NaCl, 20 mM CaCl₂ and 0.1% Brij35 (manufactured by ICI), 10 µl of a 0.1% type V collagen (a pepsin-digested product derived from bovine placenta, manufactured by YAGAI Corporation) solution, 5 µl of the sample obtained in Section (1) (a preparation of 0.01 to 2.0% aqueous solution), and 10 µl of an MMP-2 or MMP-9 solution, and allowed to enzymatically react at 37°C for 24 hours. A negative control (NC) was used which contained neither the sample nor MMP-9 but contained the 50 mM Tris-HCI buffer in place of them to have the same amount. A positive control (PC) was also used which contained no sample but the 50 mM Tris-HCI buffer in place of it to have the same amount. After the reaction, 20 µl of 0.5 M ethylenediaminetetraacetic acid (pH 7.5), 5 µl of 10% sodium dodecyl sulfate, and 5 µl of 0.1% bromophenol blue were added thereto and heated with hot water. 10 µl of the mixture was subjected to SDS-PAGE and then transferred to a PVDF membrane according to western blotting, in which bands of V type collagen and its decomposition products were separated and identified using POD Immnostain set (manufactured by Wako Pure Chemical Industries, Ltd.). Each band density was measured with a densitometer, and the abundance ratio (IntOD%: S') of the decomposition product band chain (α1' or α2' chain) to α1 or α2 chain of V type collagen was determined. The abundance ratio (PC') of the decomposition product in the positive control (PC) was normalized as 100, and the decomposition-inhibition rate was calculated by subtracting each ratio from 100 (Fig. 3).

Fig. 3 indicates that the shark cartilage extract obtained in Section (1) inhibits the MMP-2 activity and the MMP-9 activity in a concentration-dependent manner and that the activity is inhibited by about 90% at a concentration of 2%.

### (7) Fractionation by Isoelectric Focusing

In 50 ml of distilled water was dissolved 0.5 g of the shark cartilage extract obtained in Section (1) and run at 12 W for 120 minutes in an isoelectric focusing system Rotofor (manufactured by Bio-Rad Laboratories, Inc.). Each fraction was measured for pH and examined for MMP-9-inhibiting activity in the same manner as in Section (6) (Fig. 4).

As shown in Fig. 4, the MMP-9-inhibiting activity was higher in fractions with acid isoelectric points and particularly higher in pH 2.0-3.0 fractions.

### (8) Fractionation by Superdex® 75 gel filtration HPLC

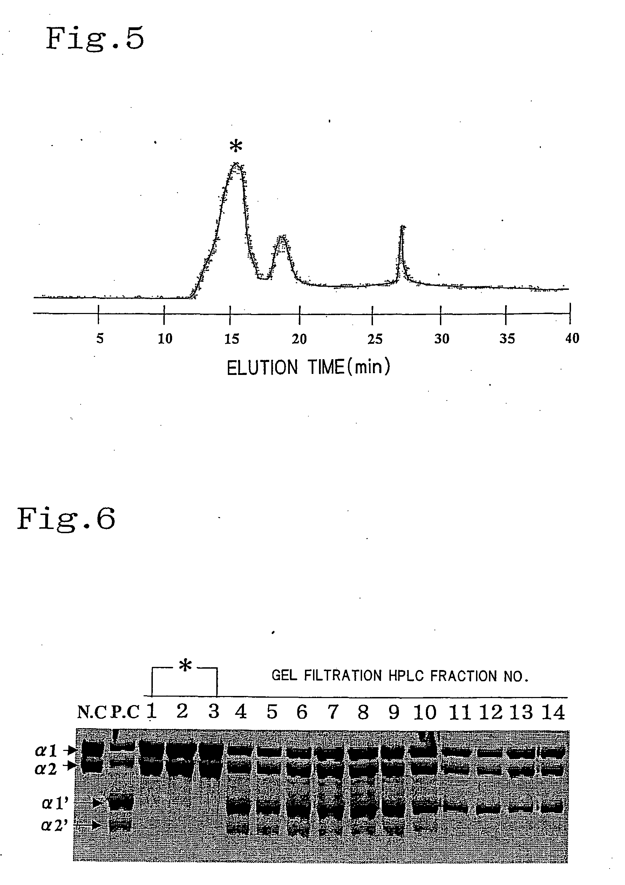

Fractions with high MMP-9-inhibiting activity as a result of the isoelectric focusing of Section (7) were centrifuged (12,000 rpm, 30 minutes). The resulting supernatant was filtrated through a 0.45 µm filter, and then 200 µl of the filtrate was fractionated by gel filtration HPLC. The separation was performed using the conditions of an eluting solution of 50 mM Tris-HCI (pH 7.86) containing 200 mM NaCl and 10 mM CaCl₂ and a column of Superdex® 75 (manufactured by Amersham Biosciences). The detection was performed at a flow rate of 0.5 ml/min and a wavelength of 230 nm (Fig. 5). Using FRACTION COLLECTOR (manufactured by Bio-Rad Laboratories, Inc.), a fraction was obtained every minute from 10 minutes immediately before a peak arose, and then each fraction was examined for MMP-9-inhibiting activity in the same manner as in Section (6) (Fig. 6).

Figs. 5 and 6 show that the MMP-9-inhibiting activity is present in macromolecular fractions with molecular weights of 100 kDa or more (marked with an * in the drawings).

### (9) Cellulose Acetate Membrane Electrophoresis

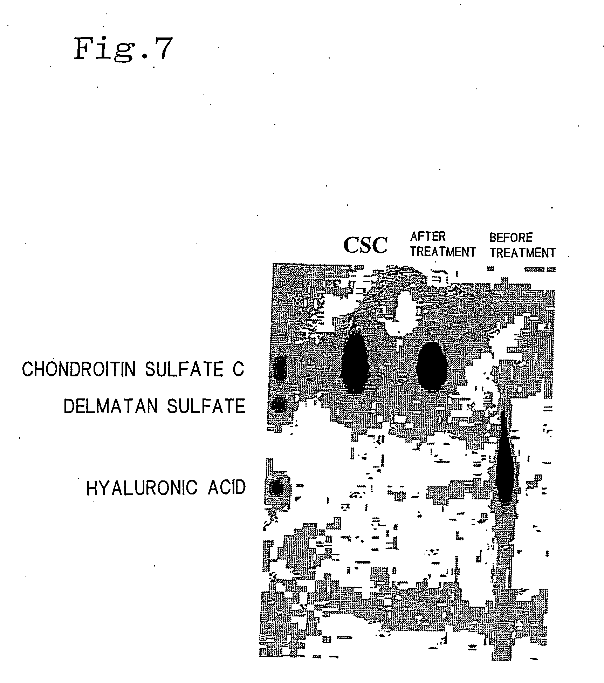

Thirty parts by volume of 0.5 N NaOH was added to one part by volume of the fraction with the MMP-9-inhibiting activity and allowed to react at 4°C for 24 hours. Neutralization was then performed with 1 N HCl, and 4.5 ml of a pronase buffer was added and thermal denaturation was performed (100°C, 10 minutes). After cooling to 50°C, thymol was added. The mixture was then allowed to react at 50°C for 48 hours. In order to remove proteins, 4.5 ml of a 30% trichloroacetic acid solution was added and allowed to react at 4°C for 1 hour. The solution was centrifuged (8,000×g, 15 minutes), and the resulting supernatant was dialyzed against distilled water and lyophilized. The dried product was subjected to electrophoresis with a cellulose acetate membrane. The electrophoresis was performed using a pyridine-formic acid buffer containing 0.1 M pyridine and 0.47 M formic acid. The markers used were chondroitin sulfate C, dermatan sulfate, and hyaluronic acid (Fig. 7).

Fig. 7 shows that the macromolecular fraction with the MMP-9-inhibiting activity was detected as a broad band before the pronase treatment but detected at the same position as chondroitin sulfate C (CSC) after the pronase treatment. Thus, it has been demonstrated that the acid, hydrophilic, macromolecular fraction with the MMP-9-inhibiting activity contains chondroitin sulfate C and is bonded to a protein.

### (10) Fractionation by Superdex® 200 Gel Filtration HPLC

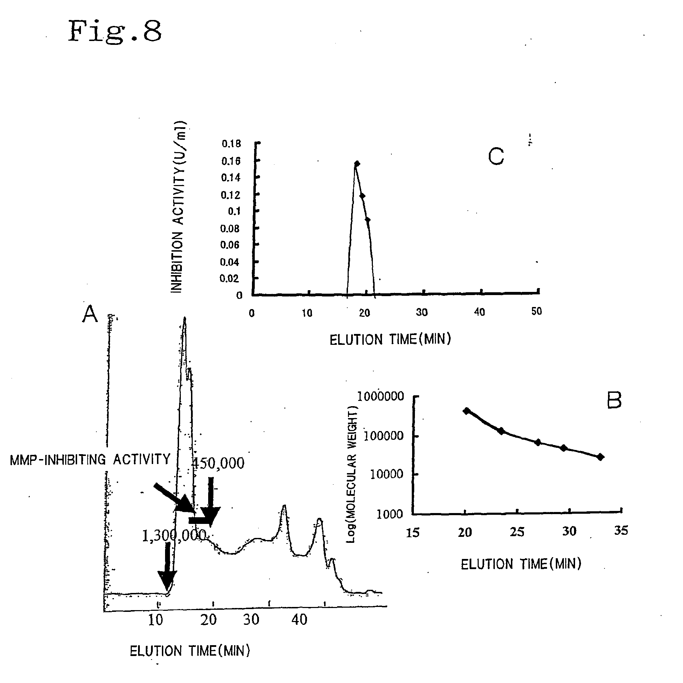

The shark cartilage extract obtained in Section (1) was dissolved in distilled water to form a 1 mg/ml solution. The solution was centrifuged (12,000 rpm, 30 minutes), and the resulting supernatant was filtrated through a 0.45 µm filter, and then 200 µl of the filtrate was fractionated by gel filtration HPLC. The separation was performed using the conditions of an eluting solution of 50 mM Tris-HCl (pH 7.86) containing 200 mM NaCl and 10 mM CaCl₂ and a column of Superdex® 200 (manufactured by Amersham Biosciences). The detection was performed at a flow rate of 0.5 ml/min and a wavelength of 230 nm (Fig. 8). Using FRACTION COLLECTOR (manufactured by Bio-Rad Laboratories, Inc.), a fraction was obtained every minute, and then each fraction was examined for MMP-9-inhibiting activity in the same manner as in Section (6).

Fig. 8 shows that the MMP-9-inhibiting activity is present in macromolecular fractions with molecular weights of 500 kDa or more.

### Comparative Example 1

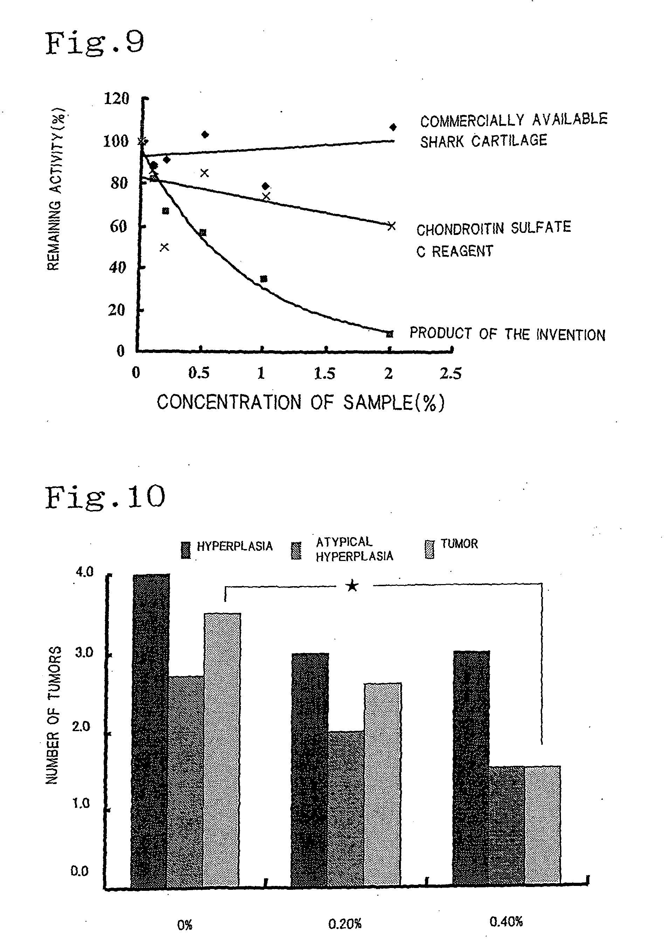

The acid, hydrophilic, macromolecular fraction obtained in Section (8) of Example 1 (the product of the invention), chondroitin sulfate C, and commercially available shark cartilage were examined for MMP-9-inbihiting activity by the method described in Section (6) of Example 1. The results are shown in Fig. 9.

Fig. 9 shows that chondroitin sulfate C and commercially available shark cartilage each have little MMP-9-inhibiting activity. For the purpose of comparing the components of the product of the invention with those of commercially available shark cartilage, the amounts of chondroitin sulfate and amino acids were determined of each of them. As a result, there was no significant difference in the amount of chondroitin sulfate, but the amount of amino acids was remarkably larger in the product of the invention. Since chondroitin sulfate C alone has little MMP-9-inhibiting activity, it is suggested that the MMP-9-inhibiting activity should be present in protein parts rather than in sugar chains.

### Example 2

### Effects of Ingested Shark Cartilage Extract on Tumor-Bearing Hamsters

### (1) Preparation of Experimental Diet

CE-2 powder purchased from CLEA Japan, Inc. was used as a basal diet. To the basal diet was added 0.2% or 0.4% by weight (hereinafter sometimes abbreviated as "%") of the shark cartilage extract prepared in Section (1) of Example 1, and the mixture was used as an experimental diet. The hamsters as described below were freely fed on the basal diet or the experimental diet and water. Each hamster fed on the experimental diet was examined for weight and the amount of diet intake. Between the same groups, there was found no significant difference in weight or the amount of diet intake.

### (2) Experimental Animals

Syrian golden hamsters (6-week old) were purchased from Nippon SLC Co., Ltd. Four or five hamsters were housed in an animal room of a plastic cage controlled at a room temperature of 24±1°C and a humidity of 60±5% on a 12:12-hr light:dark cycle. After housed, the animals were fed with no experiment for one week to adapt the environment. The animal experiments were performed in Institute of Laboratory Animals of Nara Medical University according to the principles of morality in animal experiments for the institute.

### (3) Hamsters with Pancreatic Duct Cancer Induced by N-nitrosobis(2-oxopropyl)amine (BOP)

50 mg/kg weight of BOP was subcutaneously administered to each animal, and according to a short-term pancreatic cancer development system (Mizumoto K. et al., J. Natl. Cancer Inst. 80:1546-57, 1988), a series of treatment was performed twice which included administration of choline-devoid diet, intraperitoneal administration of 500 mg/kg weight of ethionine, intraperitoneal administration of 800 mg/kg weight of methionine, and subcutaneous administration of 20 mg/kg weight of BOP, so that pancreatic duct cancer-inducing hamsters were produced. The experimental diet was fed from 50 days to 100 days after the experiment was started (Naho Murata, "Effect of Water Extract of Bovine and Shark Cartilage on Development of Pancreatic Duct Cancer in Hamsters," Journal of Nara Medical Association, 53 (5,6), 241-52, 2002).

During the experimental period, there was found no difference in weight between the respective groups or found no significant difference in final body weight, the weight of pancreas or liver, or the amount of diet intake. During the experimental period, no animal died except that immediately after feeding of the experimental material was started, one animal died in the 0.4% shark cartilage diet group. The general conditions of the animals such as hair gloss were better in the 0.4% group than in the other groups.

All the animals that were fed on the experimental diet for 50 days and alive on the 100th day were sacrificed and autopsied. Fig. 10 shows the numbers of pancreatic cancer lesions and pancreatic duct cancers in the autopsied animals. It was found that the incidence per animal of pancreatic duct epithelium hyperplasia or atypical hyperplasia tended to decrease in the 0.2% or 0.4% group but was not significantly different between the 0.2 or 0.4 % group and the basal diet group. The incidence of pancreatic duct cancers was 3.1±2.0 in the basal diet group, 2.6±2.1 in the 0.2% group, and 1.4±0.9 in the 0.4% group. It was found that the incidence of pancreatic duct cancers tended to decrease in correlation with the dose of the shark cartilage extract. The incidence of pancreatic duct cancers was significantly lower in the 0.4% group than in the basal diet (0%) group (the star indicates P<0.05 in the drawing).

### (4) Pancreatic Cancer Tissue-Implanted Hamsters

N-nitrosobis(2-hydroxypropyl)amine (BHP)-induced pancreatic cancer tissue, which was serially transplanted into hamsters at two portions of both sides of posterior back and well differentiated, was implanted using a metal trocar. Before the experimental diet was administered, a waiting period of one week was provided for cancer establishment in order that the effect of inhibiting tumor growth should be observed rather than the effect of inhibiting establishment of the implanted cancer. The experimental diet was then administered for 3 weeks. In the observation of tumor growth based on ellipse approximation of tumor shape, major and minor axes were measured, and the volumes of tumors were calculated from the formula: 4/3×π×(major axis/2)x(minor axis/2)2 and compared. Some no tumor-bearing hamsters were fed on the experimental diet and used as part of the experiment.

As a result, in 3 weeks after the tumor was established, the tumor growth was inhibited in the experimental diet group in contrast to the basal diet group.

### (5) Collection of Blood Serum and Tissues

After the experimental period was completed, whole blood was collected from the abdominal aorta of the hamsters under ether anesthesia and allowed to stand at room temperature to form a supernatant. The separated supernatant was centrifuged (3,000 rpm, 20 minutes) to give blood serum. Tumor tissues and non-tumor tissues were also collected as needed, and frozen and stored at -80°C. MMP was extracted from the tissues by a method including the steps of adding 500 µl of a 50 mM Tris-HCI buffer containing 1% Tween and 300 mM NaCl to 10 mg of the tissue, homogenizing the tissue in a microtube, incubating the tube for 15 minutes in ice, and then performing centrifugation at 3,000 rpm for 30 minutes to form a supernatant for use as a tissue extract. All the experimental processes were performed under sterile conditions.

### (6) Measurement of MMP-9-Inhibiting Activity of Blood Serum of No Tumor-Bearing or Tumor-Bearing Hamster Taking Shark Cartilage Extract

A 100 mM Tris-HCl buffer (pH 7.86) was prepared which contained 400 mM NaCl, 20 mM CaCl₂, and 0.1% Brij35, and this buffer was diluted 2-fold to form a 50 mM Tris-HCl buffer (pH 7.5). The blood serum was diluted to an appropriate concentration (5- to 20-fold) with the 50 mM buffer and then subjected to measurement of the MMP-inhibiting activity.

To a 0.6 ml microtube were added 10 µl of a 100 mM Tris-HCl buffer (pH 7.86) containing 400 mM NaCl, 20 mM CaCl₂, and 0.1% Brij35, 10 µl of a 0.1% type V collagen (a pepsin-digested product derived from bovine placenta, manufactured by YAGAI Corporation) solution, 5 µl of the blood serum sample, and 10 µl of an MMP-9 solution, and allowed to enzymatically react at 37°C for 24 hours. A negative control (NC) was used which contained neither the blood serum nor MMP-9 but contained the 50 mM Tris-HCl buffer in place of them to have the same amount. A positive control (PC) was also used which contained no blood serum but the 50 mM Tris-HCl buffer in place of it to have the same amount. After the reaction, 20 µl of 0.5 M ethylenediaminetetraacetic acid (pH 7.5), 5 µl of 10% SDS, and 5 µl of 0.1% BPB were added thereto and heated with hot water. 10 µl of the mixture was subjected to SDS-PAGE and then transferred to a PVDF membrane according to western blotting, in which bands of V type collagen and its decomposition products were separated and identified using POD Immnostain set (manufactured by Wako Pure chemical Industries, Ltd.). Each band density was measured with a densitometer, and the abundance ratio (IntOD%: S') of the decomposition product band chain to a 1 or a2 chain of V type collagen was determined. The abundance ratio (PC') of the decomposition product in the positive control (PC) was normalized as 100, and the decomposition-inhibition rate was calculated by subtracting each ratio from 100. Using the dilution factor (D') and the amount of the application (A'), the inhibition activity was also expressed as per ml of blood serum.

### Results

When the no tumor-bearing hamsters were fed on the experimental diet (0.4%) or the basal diet for 2 weeks, the activity (U/ml) of inhibiting V type collagen-decomposing MMP-9 in blood serum was 4.95±0.06 in the experimental diet group and 4.13±0.59 in the basal diet group. It was found that the MMP-9-inhibiting activity tended to increase when the shark cartilage extract was ingested.

When the tumor-bearing hamsters were fed on the experimental diet (0.4%) or the basal diet for 3 weeks, the activity (U/ml) of inhibiting V type collagen-decomposing MMP-9 in blood serum was 4.1±0.3 in the experimental diet group and 3.2±0.7 in the basal diet group.

These indicate that the MMP-9-inhibiting activity decreased from 4.1 U/ml to 3.2 U/ml by about 20% due to carcinogenesis but was significantly restored by the intake of the shark cartilage extract to be close to that of the no tumor-bearing hamsters. It was also found that the MMP-9-inhibiting activity increased by about 20% in the healthy no tumor-bearing hamsters that ingested the shark cartilage extract.

### Example 3

### Amount of Haptoglobin in Blood Serum of Hamsters

No tumor-bearing hamsters were fed on the shark cartilage extract for 2 weeks, and then their blood serum was separated by SDS-PAGE and stained with CBB. As a result, a band of about 80 kDa increased in the experimental diet group relatively to that in the basal diet group. The 80 kDa band was cut out and subjected to structural analysis (with a protein sequencer PPSQ 21 manufactured by Shimadzu Corporation). As a result, it was found that the 80 kDa band corresponded to endogenous haptoglobin of the hamster (a chain: VDLSNDAMDTADDS (SEQ ID NO: 1), β chain: IIGGSLDAKGSFPW (SEQ ID NO: 2)).

Haptoglobin has the function of biding hemolysis-induced oxidized hemoglobin and neutralizing oxidative angiopathy toxicity and is used as a maker protein not only for haemolysis but also for many diseases such as malignancy of cancer recently (Pathol Oncol Res. 1998;4(4):271-6; Br J Cancer. 1991 Aug;64(2):386-90). It is also reported that haptoglobin has the ability to inhibit cathepsin B (Can J Biochem. 1982 Jun;60(6):631-7) which has been suggested to have a relation with cancer invasion and metastasis (Chin Med J (Engl). 1998 Sep; 111(9):784-8; FEBS Lett. 1999 Jul 23;455(3):286-90) and that a haptoglobin-hemoglobin complex can induce apoptosis of cancer cells (Scand J Clin Lab Invest. 1995 Oct;55(6):529-35). At present stage, it is difficult to provide a clear interpretation on the effect on haptoglobin. A possible interpretation is that the ingested shark cartilage extract could increase haptoglobin in blood serum so that hepatic function could remain good.

### Example 4

### Effect of Ingested Shark Cartilage Extract on Cancer Patients

2 g of the shark cartilage extract prepared in Section (1) of Example 1 was orally administered to a male volunteer (50's) suffering from pancreatic cancer every day for 4 months. His blood serum was analyzed before and after the intake. No other anti-cancer agent was administered to the patient, because such an agent should have had a great side effect.

### (1) Measurement of MMP-9-Inhibiting Activity in Blood Serum of Tumor-Bearing Patient Ingesting Shark Cartilage Extract

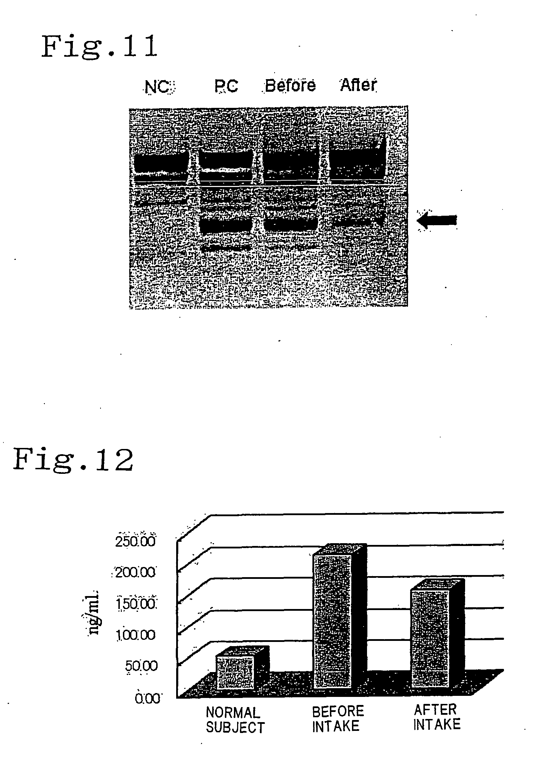

The blood serum of the patient was diluted 20-fold and then measured for MMP-9-inhibiting activity in the same manner as in Section (6) of Example 2. The results are shown in Fig. 11. From a comparison between NC (Negative Control: a V type collagen substrate solution only) and PC (Positive Control: a V type collagen substrate solution and MMP-9 added thereto and incubated), it is apparent that the lane "PC" has low-molecular-weight V type collagen as a decomposition product by MMP-9 (indicated by the arrow in the drawing).

The decomposition product is observed in the lane "Before" (blood serum of the patient before the intake of the shark cartilage extract) similarly in the lane "PC", while the decomposition product is clearly reduced in the lane "After" (after the 4-month intake of the shark cartilage extract). Thus, it has been demonstrated that the MMP-9-inhibiting activity increases in the cancer patient ingesting the shark cartilage extract.

### (2) Measurement of the Amount of Type IV Collagen

### Decomposition Product in Pancreatic Cancer Patient by ELISA Method

If the MMP-9 or 2 activity is high in a cancer patient, type IV collagen will be decomposed so that its fragments can be increased in blood serum. Thus, the MMP-9 activity was evaluated by measurement of the amount of fragments of type IV collagen in blood serum with an ELISA measurement kit, Panassay IV-C (manufactured by DAIICHI Fine Chemical Co., Ltd).

Blood serum was collected from the patient before and after the intake of the shark cartilage extract. Blood serum was also collected from three healthy subjects as a control. To a test tube were added 50 µl of 20-fold diluted blood serum and 300 µl of the enzyme-labeled antibody solution. Antibody binding beads were then added to the test tube, mixed and then allowed to stand for 1 hour. After the standing, a cleaning liquid was added to the test tube, and the reaction liquid was removed by aspiration with an aspirator, and this process was performed 4 times. To the remaining beads were added 300 µl of a liquid coloring reagent and 100 µl of a substrate solution, mingled and then allowed to stand for 30 minutes. After the standing, 1 ml of a quencher solution was added, and the absorbance of the mixture was measured at a wavelength of 450 nm with water as a control. The amount of fragments of type IV collagen in blood serum was determined from the absorbance based on a previously prepared calibration curve. The results are shown in Fig. 12 ("Normal Subject" indicates the average value of the amounts of the three healthy subjects).

The amount of IV collagen in blood serum is significantly higher in the pancreatic cancer patient before the intake of the shark cartilage extract than in the normal subject. Thus, it is suspected that type IV collagen can undergo decomposition so that invasion, metastasis, or growth of the cancer can occur. After the intake of the shark cartilage extract, the amount has steadily decreased. This also suggests that the ingested shark cartilage extract can be effective in inhibiting the MMP activity.

### INDUSTRIAL APPLICABILITY

From a comparison with an experiment of pancreatic cancer inhibition using an synthetic MMP inhibitor OPB-3206 in a prior report (Carcinogenesis. 1999 Jul;20(7):1323-9), it is apparent that in the experiment of pancreatic cancer inhibition, the proteoglycan of the invention is as effective as the synthetic inhibitor in inhibiting cancer. In addition, the shark cartilage-derived product of the invention does not have significant side effects such as a decrease in the weigh or mobility of experimental animals, which are specific to synthetic MMP inhibitors, and thus it is a hopeful cancer-development-inhibiting material available from natural products. The proteoglycan having such characteristics and a clear mechanism can easily be produced by the method of producing the proteoglycan according to the invention.

## Claims

1. An isolated proteoglycan which is derived from a water extract of cartilage of cartilaginous fish and whose main component has a molecular weight of 500 kDa or more.

2. The proteoglycan of Claim 1, wherein it is insoluble in an alcohol.

3. The proteoglycan of Claim 1 or 2, wherein it has a glycosaminoglycan part mainly composed of chondroitin sulfate C.

4. The proteoglycan of any one of Claims 1 to 3, wherein it has a matrix metalloprotease-inhibiting activity.

5. The proteoglycan of Claim 4, wherein the matrix metalloprotease is MMP-9, and the inhibiting activity is an effect of canceling a reduction in an MMP-9-inhibiting activity in the blood serum of a tumor-bearing animal fed on a 0.4% by weight-product-containing feed or an effect of increasing, by at least 5%, an MMP-9-inhibiting activity in the blood serum of a tumor-bearing animal fed on a 0.4% by weight-product-containing feed.

6. The proteoglycan of any one of Claims 1 to 5, wherein it has an effect of increasing a cathepsin B-inhibiting activity when taken in an effective amount into a living body.

7. The proteoglycan of any one of Claims 1 to 6, wherein it has an activity of increasing the amount of haptoglobin in blood serum when taken in an effective amount into a living body.

8. A composition comprising the proteoglycan of any one of Claims 1 to 7.

9. The composition of Claim 8, wherein it is for use in an improvement in quality of life.

10. A pharmaceutical composition, comprising the proteoglycan of any one of Claims 1 to 7 as an active ingredient.

11. A method of producing the proteoglycan of any one of Claims 1 to 7, comprising the steps of:
pulverizing cartilaginous fish-derived cartilage into a pulverized product with an average particle diameter of 100 µm or less;
adding water to the pulverized product and extracting water-soluble components from it;
separating an aqueous phase that contains the extracted water-soluble components; and
adding an alcohol to the aqueous phase to produce a precipitate.
